(19) Europäisches Patentamt
European Patent Office
Office européen des brevets

(11) Veröffentlichungsnummer: **0 276 729**
**A2**

(12) # EUROPÄISCHE PATENTANMELDUNG

(21) Anmeldenummer: **88100708.2**

(22) Anmeldetag: **20.01.88**

(51) Int. Cl.[4]: **C12N 1/20**, C02F 3/34, //(C12N1/20,C12R1:00)

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 3954.

(30) Priorität: **24.01.87 DE 3702117**

(43) Veröffentlichungstag der Anmeldung: **03.08.88 Patentblatt 88/31**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lingens, Franz, Prof. Dr.**
**Im Asemwald 32/4**
**D-7000 Stuttgart 70(DE)**
Erfinder: **Voelskow, Hartmut, Dr.**
**Akazienstrasse 22**
**D-6234 Hattersheim am Main(DE)**

(54) **Verfahren zum Abbau aromatischer Verbindungen mit Hilfe von Rhodococcus rhodochrous.**

(57) Rhodococcus rhodochrous DSM 3954 besitzt die Fähigkeit chlorierte und/oder nicht-chlorierte Aromaten einzeln oder in Gemischen als Kohlenstoffquelle zu verwerten und abzubauen.

EP 0 276 729 A2

Xerox Copy Centre

## Verfahren zum Abbau aromatischer Verbindungen mit Hilfe von Rhodococcus rhodochrous

Bakterien, die in der Lage sind, aromatische Verbindungen abzubauen, kommen im Erdboden häufig vor. Auch zum Abbau chlorierter Aromaten konnten bereits verschiedene Kulturen eingesetzt werden [Vandenbergh P.A. et al., Appl Env. Microbiol. 42, 737 (1981); You J.S. et al., J. Agric. Food Chem. 30, 274 (1982); Zeyer J., Kearney P.C., Pesticide Biochem. Biophys. 17, 224 (1982).]

Das Wachstum der Bakterien auf chlorierten Aromaten ist in der Regel sehr langsam. Der Abbau von 500 ppm dieser Substanzen dauert 7 Tage bis 3 Wochen. Selten ist ein Bakterium in der Lage gleich mehrere chlorierte Aromaten zu spalten.

Es wurde nun überraschend gefunden, daß das Bakterium Rhodococcus rhodochrous, das bei der Deutschen Sammlung von Mikroorganismen unter der Nummer DSM 3954 hinterlegt ist, die Fähigkeit besitzt, chlorierte und/oder nicht-chlorierte Aromaten einzeln oder in Gemischen als Kohlenstoffquelle zu verwenden und abzubauen. Es wurde weiterhin festgestellt, daß dieser Mikroorganismus diese Stoffe - schneller verwertet als andere Aromatenabbauer.

Die Erfindung betrifft somit ein Verfahren zum Abbau aromatischer Verbindungen, das dadurch gekennzeichnet ist, daß die aromatischen Verbindungen mit bis zu 22 C-Atomen einzeln oder in Gemischen miteinander mit Hilfe von Rhodococcus rhodocrous DSM 3954 abgebaut werden, ausgenommen Toluidin als einzelne abzubauende aromatische Verbindung.

Im folgenden wird die Erfindung im einzelnen, insbesondere in den bevorzugten Ansprüchen, beschrieben. Ferner wird die Erfindung in den Patentansprüchen definiert.

Rhodococcus rhodochrous DSM 3954 wurde aus seiner Ackererdprobe isoliert. Eine Charakterisierung des Stammes ist in FEMS Microbiology Letters 24, 123-126 (1984) aufgeführt.

Nicht nur der Stamm DSM 3954 ist zum Abbau von Aromaten geeignet, sondern auch dessen Varianten und Mutanten.

Das Bakterium kann eine Vielzahl von Aromaten abbauen. Geeignet sind aromatische Verbindungen mit bis zu 22 Kohlenstoffatomen, die beispielsweise unter Verbindungen der allgemeinen Formel I fallen,

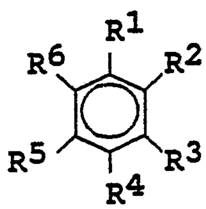

in der $R^1$ bis $R^6$ unabhängig voneinander Wasserstoff, Methyl, Hydroxymethyl, Phenyl, Hydroxyl, Carbonyl und Chlor sein können, wobei der Phenylrest ebenfalls mit den genannten Gruppen substituiert sein kann.

Bevorzugt wird der Mikroorganismus zum Abbau von Aromaten mit bis zu 12 Kohlenstoffatomen eingesetzt. Insbesondere bevorzugt abgebaut werden Anilin und seine Derivate, wie beispielsweise Chloranilin, Dichloranilin, Chlormethylanilin, Hydroxymethylanilin und Methylanilin, Toluol und seine Derivate, wie z.B. Chlortoluol, Benzoesäure und seine Derivate, wie beispielsweise Chlorbenzoesäure, Hydroxybenzoesäure, Chlorhydroxybenzoesäure, Di-und Trihydroxybenzoesäure sowie Aminobenzoesäure, Aminohydroxybenzoesäure und Chloraminobenzoesäure, Benzylalkohol und seine Derivate, wie z.B. Hydroxybenzylalkohol und Aminobenzylalkohol, Brenzkatechin und seine Derivate, wie z.B. Methylbrenzkatechin und Chlorbrenzkatechin, und auch Biphenyl und seine Derivate, wie z.B. Chlorbiphenyl.

Die genannten Verbindungen können einzeln oder in Gemischen von dem Stamm DSM 3954 verwertet werden. Auch Chloraromaten unterschiedlicher Derivate können einzeln oder in Gemischen erfindungs gemäß gut abgebaut werden.

Es ist vorteilhaft Rhodococcus rhodochrous vor der Verwendung zum Abbau von Aromaten kurz an diese C-Quellen zu adaptieren, insbesondere dann, wenn der Mikroorganismus längere Zeit für solche Anwendungen nicht benutzt wurde. Dazu verfährt man nach dem in der Deutschen Offenlegungsschrift 34 17 443 beschriebenen Verfahren zur Adaptation von Mikroorganismenkulturen an biologisch schwer abbaubare Aromaten. Gemäß diesem Verfahren wird Rhodococcus rhodochrous unmittelbar an die abzubauenden höher substituierten Aromaten adaptiert. Ein geeignetes Mineralmedium wird mit 100 bis 500 ppm seiner oder mehrerer der mehrfach substituierten aromatischen Verbindungen versetzt, mit einer Kultur des Stamms DSM 3954 beimpft und unter Schütteln bei 20 bis 35°C über seinen Zeitraum von 2 bis 8

Tagen inkubiert. Schon während dieser Adaptation erreicht der Mikroorganismus nach 1 bis 2-maligem Überimpfen eine Zelldichte (OD 580) von durchschnittlich 0,5 bis 4. Inkubationszeit und Zelldichte hängen von den verwendeten Aromaten ab und können daher auch etwas von den obengenannten Werten abweichen.

Für die eigentliche Hauptkultur verwendet man wieder ein für Rhodococcus rhodochrous geeignetes Mineralmedium, dem man je 0,01 mM bis mM, bevorzugt 0,1 mM bis 1 mM, der Chlor enthaltenden Aromaten und/oder 0,01 mM bis 10 mM, bevorzugt 0,5 bis 5 mM, der chlorfreien Aromaten zugibt. Etwa folgende Summen-Konzentrationen sollten in der Kulturlösung nicht überschritten werden:
Chlorierte Aromaten 2 mM
Chlorfreie Anilin bzw. Toluidinderivate 5 mM
Chlorfreie Phenolderivate 5 mM
Benzoesäure, Benzylalkohol und Derivate 10 mM
Die Gesamtkonzentration an Aromaten sollte nicht über 10 mM liegen. Die angegebenen Werte sind Richtwerte, oberhalb derer mit verlangsamtem Wachstum zu rechnen ist. Durch entsprechende Nachdosierung ist jedoch auch ein kontinuierlicher Abbau, auch größerst Mengen, möglich.

Ein derartiges Nährmedium wird mit Rhodococcus rhodochrous DSM 3954 beimpft und aerob, bevorzugt auf einem Rundschüttler, bei 16 bis 35°C, vorzugsweise bei 20 bis 30°C inkubiert. Die Geschwindigkeit des Abbaus ist von den einzelnen aromatischen Komponenten in der Nährlösung abhängig.

Die genannten Aromaten können der Nährlösung als alleinige Kohlenstoffquelle zugegeben werden. Sie können aber auch zusammen mit leichter verwertbaren C-Quellen zugesetzt werden, wodurch unter Umständen eine verbesserte Zellvermehrung hervorgerufen werden kann.

Geeignete leichter verwertbare C-Quellen sind beispielsweise Zucker. z.B. Glucose, Zuckeralkohole, wie z.B. Mannit oder Sorbit, Paraffine, aliphatische Alkohole und Carbonsäuren mit jeweils bis zu 18 C-Atomen. Aceton oder Dimethylsulfoxid. Die leichter verwertbare C-Quelle ist dem Nahrmedium zusammen mit den abzubauenden aromatischen Verbindungen oder auch bevorzugt 1 bis 3 Tage bevor diese zugegeben werden zuzusetzen.

Die Konzentration der leichter abzubauenden C-Substrate kann in einem für Wachstumsmedien üblichen Bereich liegen, bevorzugt bei 2 bis 10 g/l Nährmedium.

Das Wachstum von Rhodococcus rhodochrous DSM 3954 ist auf den genannten Aromaten überdurchschnittlich gut. Während bekannte Aromatenabbauer auf substituierten Aromaten sehr langsam wachsen und auf Chloraromaten innerhalb von 7 Tagen nur OD-Werte von 0,4 bis 0,6 erreichen, konnten bei Verwendung von DSM 3954 und Chloraromaten als alleiniger C-Quelle nach 4 bis 7 Tagen OD-Werte von > 1 gemessen werden.

Insbesondere der Abbau von Chlormethylanilin ist mit Hilfe von Rhodococcus rhodochrous DSM 3954 in kürzerer Zeit möglich als bei bisher beschriebenen Mikroorganismen. Die beschriebene Mischkultur V-8749 [Voelskow H., Vom Wasser 63, 87, (1984)] benötigt zum Abbau von 150 mg Chlormethylanilin pro Liter Nährmedium 7 Tage. Eine adaptierte Kultur von DSM 3954 kann die gleiche Menge dieser Verbindung in weniger als 5 Tagen abbauen.

Im folgenden wird die Erfindung anhand von Beispielen detailliert erläutert. Prozentangaben beziehen sich auf das Gewicht, wenn nicht anders angegeben.

Beispiel

1. Der Stamm DSM 3954 wurde auf Agarmedium PM mit 500 mg/l (500 ppm) o-Toluidin als C-Substrat 7 Tage vorkultiviert.

Zusammensetzung des Mediums PM: 0,5 g $MgSO_4$ • 7 $H_2O$, 1,5 g $(NH_4)_2SO_4$, 5,0 g $KH_2PO_4$, 0,33 g $CaCl_2$, 10 ml Spurenelemente SPV-11, $H_2O$ ad 1 l;
Zusammensetzung der Spurenelementlösung SPV-11: Fe (III) citrate 4,1 mM, $MnSO_4$ 1,3 mM, $ZnCl_2$ 0,73 mM, CaCl 20 mM, $CoCl_2$ 0,03 mM, $Na_2B_4O_7$ 0,05 mM, $Na_2MoO_4$ 0,04 mM.

Von der Kultur wurde eine Suspension in physiologischer NaCl-Lösung hergestellt (2 Impfösen der Plattenkultur auf 1 ml Lösung) und 1 ml auf 100 ml Flüssigmedium PM mit je 0,5 mM der einzelnen Aromaten oder Chloraromaten überimpft. Die Aromaten wurden vor Zugabe in Ethanol vorgelöst.

Als Kulturgefäße dienten 100 ml-Erlenmeyerkolben mit 75 ml Füllung oder 300 ml-Erlenmeyerkolben mit 150 bis 200 ml Füllung. Die Inkubation erfolgte auf einem Rundschüttler bei 27°C bis 30°C und 110 Rpm. Die Probenahme geschah regelmäßig nach 4 bis 8 Tagen Kulturdauer und bei Bedarf auch weiter nach 10, 14, 18 und 23 Tagen. 1 bis 2 ml der Kulturbrühe wurden klarzentrifugiert und für analytische Dünnschichtchromatographie bei pH 1 bis 2 mit der doppelten Menge Essigester ausgeschüttelt und

vakuumgetrocknet. Einige Kulturproben wurden insgesamt, d.h. nicht abzentrifugiert, der Bestimmung des totalen organisch gebundenen Chlors (TOX) in der Abwasseranalytik nach bekannter Methode zugeführt. Einige Kontrollanalysen erfolgten auch durch GC und HPLC. In der folgenden Tabelle ist die Abbauzeit einzelner Verbindungen aufgeführt.

| aromatische Verbindung | Abbauzeit [Tage] |
|---|---|
| Anilin | 6 |
| 3-Chloranilin | 10 |
| 4-Chloranilin | 10 |
| o-Toluidin | 7 |
| m-Toluidin | 7 |
| p-Toluidin | 7 |
| 3-Chlor-2-methylanilin | 7 |
| 4-Chlor-2-methylanilin | 7 |

| aromatische Verbindung | Abbauzeit [Tage] |
|---|---|
| 5-Chlor-2-methylanilin | 7 |
| 4-Hydroxy-2-methylanilin | 14 |
| Benzoesäure | 8 |
| 4-Hydroxybenzoesäure | 8 |
| 2,3-Dihydroxybenzoesäure | 7 |
| 2,3,4-Trihydroxybenzoesäure | 14 |
| Anthranilsäure | 18 |
| 3-Aminobenzoesäure | 6 |
| 3-Amino-4-hydroxybenzoesäure | 6 |
| 5-Hydroxyanthranilsäure | 10 |
| Benzylalkohol | 7 |
| 4-Hydroxybenzylalkohol | 7 |
| 2-Aminobenzylalkohol | 18 |
| Toluol | 7 |
| 4-Chlortoluol | 7 |
| Brenzkatechin | 7 |
| 3-Methylbrenzkatechin | 10 |
| 4-Methylbrenzkatechin | 14 |
| 4-Chlorbrenzkatechin | 18 |
| Biphenyl | 18 |

Die oben genannten Verbindungen wurden im angegebenen Zeitraum vollständig abgebaut. Die folgenden Substanzen wurden im angegebenen Zeitraum teilweise abgebaut. Gutes Bakterienwachstum war in allen Fällen zu beobachten.

| aromatische Verbindung | Abbauzeit [Tage] |
|---|---|
| 2,6-Dichloranilin | 18 |
| 3-Chlorbenzoesäure | 23 |
| 4-Chlorsalicylsäure | 10 |
| 5-Chlorsalicylsäure | 23 |
| 3-Chlor-4-hydroxybenzoesäure | 23 |
| 5-Chloranthranilsäure | 18 |
| 4-Chlorbiphenyl | 8 |

2. Nach 24stündiger Voranzucht von DSM 3954 auf PM-Medium (siehe Beispiel 1) unter Zugabe von 5% Ethanol als Substrat wurde folgendes Aromatengemisch zugegeben: o-, m-, p-Toluidin, Toluol, Anilin, Benzoesäure, Phenol jeweils 1 mM.

Ansonsten wurde gemäß Beispiel 1 verfahren. Die Abbauzeit für das Aromatengemisch betrug 4 Tage.

3. Man verfuhr gemäß Beispiel 2, jedoch mit folgendem Aromatengemisch:

Anilin 2 mM
4-Chlor-2-methylanilin 0,5 mM
5-Chlor-2-methylanilin 0,5 mM
3-Chloranilin 0,25 mM
4-Chloranilin 0,25 mM

Die Abbauzeit für das Aromatengemisch betrug 7 Tage.

## Ansprüche

1. Verfahren zum Abbau aromatischer Verbindungen, dadurch gekennzeichnet, daß die aromatischen Verbindungen mit bis zu 22 C-Atomen einzeln oder in Gemischen miteinander mit Hilfe von Rhodococcus rhodochrous DSM 3954, seinen Varianten und Mutanten abgebaut werden, ausgenommen Toluidin als einzelne abzubauende aromatische Verbindung.

2. Verfahren nach Anspruch 1, dadurch gekennzeichnet, daß Verbindungen der allgemeinen Formel I abgebaut werden,

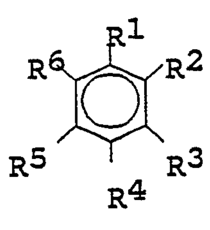

I

in der $R^1$ bis $R^6$, unabhängig voneinander Wasserstoff, Methyl, Hydroxymethyl, Phenyl, Hydroxyl, Carbonyl und Chlor bedeuten, wobei der Phenylrest ebenfalls mit den genannten Gruppen, ausgenommen Phenyl, substituiert sein kann.

3. Verfahren nach Anspruch 1 oder 2, dadurch gekennzeichnet, daß Chloraromaten abgebaut werden.

4. Verfahren nach einem oder mehreren der Ansprüche 1 bis 3, dadurch gekennzeichnet, daß dem Nährmedium eine C-Quelle zugesetzt wird, die leichter verwertbar ist als die abzubauenden aromatischen Verbindungen.

5. Verfahren nach Anspruch 4, dadurch gekennzeichnet, daß dem Nährmedium Glucose und/oder Mannit und/oder Sorbit und/oder Aceton und/oder Dimethylfsulfoxid sowie Paraffine, aliphatische Alkohole und/oder Carbonsäuren mit jeweils bis zu 18 C-Atomen zugesetzt werden.

6. Verfahren nach einem oder mehreren der Ansprüche 1 bis 5, dadurch gekennzeichnet, daß bei 18 bis 35°C kultiviert wird.

7. Rhodococcus rhodochrous DSM 3954, seiner Varianten und Mutanten, sofern sie Aromaten mit bis zu 22 C-Atomen abbauen.